(19) Europäisches Patentamt / European Patent Office / Office européen des brevets

(11) **EP 3 733 635 B1**

(12) **EUROPEAN PATENT SPECIFICATION**

(45) Date of publication and mention
of the grant of the patent:
**26.01.2022 Bulletin 2022/04**

(21) Application number: **20171606.5**

(22) Date of filing: **27.04.2020**

(51) International Patent Classification (IPC):
**B01J 29/48** (2006.01)    **C07C 29/60** (2006.01)
**C07C 33/03** (2006.01)

(52) Cooperative Patent Classification (CPC):
(C-Sets available)
**B01J 29/48; C07C 29/60; C07C 33/03;**
B01J 2229/186; B01J 2523/00          (Cont.)

(54) **SUSTAINABLE PRODUCTION OF ALLYL ALCOHOL: GAS-PHASE CONVERSION OF GLYCEROL OVER METALS PROMOTED ALUMINOSILICATE ZEOLITE CATALYSTS IN A FIXED-BED CONTINUOUS FLOW REACTOR**

NACHHALTIGE HERSTELLUNG VON ALLYLALKOHOL: GASPHASENUMWANDLUNG VON GLYCERIN ÜBER METALLGEFÖRDERTE ALUMINOSILIKAT-ZEOLITH-KATALYSATOREN IN EINEM FESTBETT-DURCHLAUFREAKTOR

PRODUCTION DURABLE D'ALCOOL ALLYLIQUE : CONVERSION EN PHASE GAZEUSE DE GLYCÉROL SUR UN CATALYSEUR DE ZÉOLITE D'ALUMINOSILICATE ACTIVÉ PAR DES MÉTAUX DANS UN RÉACTEUR À FLUX CONTINU À LIT FIXE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.04.2019 LU 101203**

(43) Date of publication of application:
**04.11.2020 Bulletin 2020/45**

(73) Proprietor: **Kemijski Institut
1000 Ljubljana (SI)**

(72) Inventors:
• **Likozar, Blaz**
  **4000 Kranji (SI)**
• **Kostyniuk, Andrii**
  **1001 Ljubljana (SI)**
• **Bajec, David**
  **8333 Semic (SI)**

(74) Representative: **Heubeck, Christian
Weickmann & Weickmann
Patent- und Rechtsanwälte PartmbB
Postfach 860 820
81635 München (DE)**

(56) References cited:
**CN-A- 102 786 499    JP-A- 2008 162 907
JP-B2- 5 095 203**

• **LAN HAI ET AL: "Synergistic Effect of Mo-Fe Bimetal Oxides Promoting Catalytic Conversion of Glycerol to Allyl Alcohol", CATALYSIS LETTERS, J.C. BALTZER, NEW YORK, vol. 147, no. 8, 1 July 2017 (2017-07-01), pages 2187-2199, XP036279400, ISSN: 1011-372X, DOI: 10.1007/S10562-017-2124-3 [retrieved on 2017-07-01]**
• **SÁNCHEZ G ET AL: "Zeolite-supported iron catalysts for allyl alcohol synthesis from glycerol", APPLIED CATALYSIS A: GENERAL, ELSEVIER, AMSTERDAM, NL, vol. 509, 20 October 2015 (2015-10-20), pages 130-142, XP029347777, ISSN: 0926-860X, DOI: 10.1016/J.APCATA.2015.09.039**

(52) Cooperative Patent Classification (CPC): (Cont.)

C-Sets
**C07C 29/60, C07C 33/03;**
B01J 2523/00, B01J 2523/15, B01J 2523/68,
B01J 2523/842

**Description**

**FIELD OF THE INVENTION**

The present disclosure relates generally but not exclusively to a novel catalyst with using tri-metallic promoted aluminosilicate zeolite catalysts for the conversion of glycerol to allyl alcohol in a gas-phase fixed-bed continuous flow reactor. In certain embodiments, methods for the conversion of glycerol to allyl alcohol with using tri-metallic promoted aluminosilicate zeolite catalysts comprising xCsyFezMo-ZSM-5 are disclosed.

**BACKGROUND OF THE INVENTION**

The depletion of fossil resources like oil, natural gas, and coal with negative impacts on climate change has inspired academic and industrial researchers to find alternative energy carriers based on renewable raw materials. Nowadays bioethanol, biodiesel, and biogas are more perspective and environmentally friendly for substitution of gasoline, diesel, and natural gas, respectively. Whereas only biodiesel production is a chemical process, based on transesterification of vegetable oils or of animal or waste fats. Biodiesel is essentially produced through transesterification of vegetable oils or animal fats by acid or basic catalysts, and glycerol (GL) is the principal by-product of the process.

Production of biodiesel grows every year, but according to stoichiometry, 1 mol of triglyceride gives 3 mol of esters and 1 mol of GL, therefore for every 9 kg of biodiesel produced about 1 kg of a crude GL by-product is formed. Almost 3.7 million tons of GL from biodiesel industry will enter on the international market by 2020. The market price of GL has dropped rapidly with the increasing of GL on the markets. Therefore, catalytic conversion of the enormous quantity of GL through biodiesel production to valuable products is an essential task and it will be a great importance for the biodiesel economy.

Allyl alcohol (AAL) is an industrially important substance, mostly used for the production of chemical intermediate in organic synthesis (glycidol, glycidyl esters, epichlorohydrin, 1,4-butanediol, allyl diglycol carbonate, amines, resins, paints, coatings, silane coupling agents, and polymer crosslinking agents), precursor of perfume, pharmaceutical, food formulations and etc. Current industrial processes for the production of AAL are fossil fuel-based routes from hydrogenation of propylene-derived acrolein. A second approach involves propylene oxide isomerization over a lithium phosphate catalyst at 300 °C with subsequent purification. In addition, the allyl alcohol can be produced by the catalyzed acetoxylation of propylene over noble metal catalysts. All these methods have many disadvantages such as non-environmentally friendly route, multistep synthesis, unsustainable process, expensive and toxic feedstocks. These disadvantages in the current industrial processes have stimulated researchers to develop and study the sustainable production of allyl alcohol from glycerol. Nowadays there is no industrial process for the large-scale preparation of allyl alcohol from glycerol. Recently, several attempts have been made and potential pathways for bio-based AAL production have been reported.

During the past few years, the one-step deoxydehydration (DODH) reaction of glycerol has received enhancing interest where typically uses heterogeneous Re catalysts (Kon Yoshihiro et al., European Patent, Application № 15306244.3, Date of publication 01.02.2017). Moreover, many homogeneous catalysts with different reductants for DODH have been published in the literature. The main disadvantages of DODH reaction are expensive catalysts, long reaction time, low feedstock concentration, liquid phase reaction, batch-type reactor, high pressure, limited recyclability, H donor and solvent are necessary.

Recently, Hai Lan et al. (Catal Lett 147 (2017) 2187-2199) studied the effect of adding Mo-Fe bimetal species to KIT-6 silica on the direct catalytic conversion of GL to AAL in the gas-phase, which has never been reported before. It was found by authors that the bimetal MoFe-x/KIT-6 catalyst showed much higher selectivity than monometallic (Fe/KIT-6 and Mo/KIT-6), which was explained by the strong synergistic effect between $Fe_2O_3$ and $MoO_3$ altering the surface moderate acid strength, surface acid amounts, and reducibility of catalysts following a hydrogen transfer mechanism. The MoFe-0.3/KIT-6 showed the highest AAL yield of 26.8 mol% without external hydrogen donors supplied to the system, which benefits from the good balance between moderate acidity and weak reducibility of catalysts. However, this catalyst exhibits a low yield of allyl alcohol and significant deactivation after time-on-stream more than 2 h.

Hence, a highly active catalyst with a better yield is still required to apply the gas-phase reaction of glycerol conversion to allyl alcohol. The gas-phase glycerol conversion reaction in a fixed-bed continuous flow reactor was chosen by us due to the glycerol conversion and allyl alcohol selectivity can be easily modified with parameters such as GHSV, temperature reaction, and catalyst weight in comparison with liquid-phase reaction.

**SUMMARY OF THE INVENTION**

The present invention solves the problems outlined above and provides the method as defined in claim 1, as well as the catalyst defined in claim 14. Preferred embodiments of the method and the catalyst are disclosed in claims from 2 to 13 and from 15 to 22 respectively, as well as in the following description.

Process for the production of allyl alcohol (AAL) comprising the steps:

(a) feeding a starting material over a heterogeneous catalyst into a fixed-bed continuous flow reactor,
(b) heating said mixture at a temperature ranging from 250 to 450 °C to obtain allyl alcohol,
(a) flowing $N_2$ as a carrier gas with total gas hourly space velocity 100-2000 $h^{-1}$,
(b) collecting the AAL product,

wherein the starting material is glycerol (GL) solution, and wherein the catalyst is a tri-metallic aluminosilicate zeolite.

The most stable yield achieved was 45.2 mol% over 5Cs2.5Fe2.5Mo-ZSM-5 catalyst after 26 h time-on-stream, which is higher and stable than results achieved in the literature data. The key parameter to the high yield for the desired allyl alcohol production is a suitable balance between acidity-basicity and reducibility behaviors of the catalyst samples. Investigation of the acid-base sites of the catalyst samples showed that the presence of a sufficient amount of Bronsted and Lewis acid and basic sites is of the crucial parameter for their catalytic performance. The invented catalyst showed the best catalytic performance in the studied reaction, most probably due to the smallest crystallite size, the highest mesopore volume and mesopore surface area, and the optimal acid and basic sites ratio.

## BRIEF DESCRIPTION OF THE FIGURES

FIG. 1    shows the proposed reaction pathways for the conversion of glycerol (GL) to allyl alcohol (AAL) in a gas-phase.

FIG. 2    shows a comparison of existing routes of gas-phase glycerol conversion into allyl alcohol through a hydrogen transfer reaction with the present work. 1 - $N_2$ gas storage tank; 2 - inlet glycerol tank; 3 - HPLC pump; 4 - convection heater, 5 - hot box; 6 - reactor; 7 - oven; 8 - packed-bed; 9 - quartz wool; 10 - steel porous plate; 11 - six port bypass valve; 12 - Peltier cooling cell; 13 - GC (Agilent GC-7890A) with FID detector and GC-MS (Shimadzu, QP2010); 14 - personal computer.

FIG. 3    shows the GC-FID chromatogram of sample after conversion of GL to AAL over the 5Cs2.5Fe2.5Mo-ZSM-5 catalysts under the following reaction conditions: catalyst, 0.5 g, TOS = 26 h; $T_{reactor}$ = 350 °C; C(GL) = 10 wt%; $GHSV_{total}$ = 625 $h^{-1}$

## DETAILED DESCRIPTION OF THE INVENTION

The present invention relates to the use of tri-metallic promoted aluminosilicate zeolite heterogeneous catalysts for the direct conversion of glycerol to allyl alcohol in a gas-phase fixed-bed continuous flow reactor.

Preferred examples of catalysts are listed in the following: ZSM-5, Mordenite, Zeolite USY, Zeolite L, Ferrierite, Zeolite Beta, Zeolite Y, Cs-Fe-Mo mixed oxides (with varying Cs/Fe/Mo molar ratios for all 3 elements, preferably from 1 to 4), Li-Fe-Mo mixed oxides (with varying Li/Fe/Mo molar ratios for all 3 elements, preferably from 1 to 4), Na-Fe-Mo mixed oxides (with varying Na/Fe/Mo molar ratios for all 3 elements, preferably from 1 to 4), K-Fe-Mo mixed oxides (with varying K/Fe/Mo molar ratios for all 3 elements, preferably from 1 to 4), Rb-Fe-Mo mixed oxides (with varying Rb/Fe/Mo molar ratios for all 3 elements, preferably from 1 to 4), Cs-Fe-Mo mixed nitrate salts (with varying Cs/Fe/Mo molar ratios for all 3 elements, preferably from 1 to 4), Li-Fe-Mo mixed nitrate salts (with varying Li/Fe/Mo molar ratios for all 3 elements, preferably from 1 to 4), Na-Fe-Mo mixed nitrate salts (with varying Na/Fe/Mo molar ratios for all 3 elements, preferably from 1 to 4), K-Fe-Mo mixed nitrate salts (with varying K/Fe/Mo molar ratios for all 3 elements, preferably from 1 to 4), Rb-Fe-Mo mixed nitrate salts (with varying Rb/Fe/Mo molar ratios for all 3 elements, preferably from 1 to 4), Cs-Fe-Mo mixed chloride salts (with varying Cs/Fe/Mo molar ratios for all 3 elements, preferably from 1 to 4), Li-Fe-Mo mixed chloride salts (with varying Li/Fe/Mo molar ratios for all 3 elements, preferably from 1 to 4), Na-Fe-Mo mixed chloride salts (with varying Na/Fe/Mo molar ratios for all 3 elements, preferably from 1 to 4), K-Fe-Mo mixed chloride salts (with varying K/Fe/Mo molar ratios for all 3 elements, preferably from 1 to 4), Rb-Fe-Mo mixed chloride salts (with varying Rb/Fe/Mo molar ratios for all 3 elements, preferably from 1 to 4), Cs-Fe-Mo mixed sulfate salts (with varying Cs/Fe/Mo molar ratios for all 3 elements, preferably from 1 to 4), Li-Fe-Mo mixed sulfate salts (with varying Li/Fe/Mo molar ratios for all 3 elements, preferably from 1 to 4), Na-Fe-Mo mixed sulfate salts (with varying Na/Fe/Mo molar ratios for all 3 elements, preferably from 1 to 5), K-Fe-Mo mixed sulfate salts (with varying K/Fe/Mo molar ratios for all 3 elements, preferably from 1 to 4), Rb-Fe-Mo mixed sulfate salts (with varying Rb/Fe/Mo molar ratios for all 3 elements, preferably from 1 to 4), Cs-Fe-Mo mixed iodide salts (with varying Cs/Fe/Mo molar ratios for all 3 elements, preferably from 1 to 4), Li-Fe-Mo mixed iodide salts (with varying Li/Fe/Mo molar ratios for all 3 elements, preferably from 1 to 4), Na-Fe-Mo mixed iodide salts (with varying Na/Fe/Mo molar ratios for all 3 elements, preferably from 1 to 4), K-Fe-Mo mixed iodide salts (with varying K/Fe/Mo molar ratios for all 3 elements, preferably from 1 to 4), Rb-Fe-Mo mixed iodide salts (with varying Rb/Fe/Mo molar ratios for all 3 elements, preferably from 1 to 4), Cs-Fe-Mo mixed fluoride salts (with varying Cs/Fe/Mo molar ratios for all 3 elements, preferably from 1 to 4), Li-Fe-Mo mixed fluoride salts (with varying Li/Fe/Mo molar ratios for all 3 elements, preferably from 1 to 4), Na-Fe-Mo mixed fluoride salts (with varying Na/Fe/Mo molar ratios for all 3 elements, preferably from 1 to 4), K-Fe-Mo mixed fluoride salts (with varying K/Fe/Mo molar ratios for all 3 elements, preferably from 1 to 4), Rb-Fe-Mo mixed fluoride salts (with varying Rb/Fe/Mo molar ratios for all 3 elements, preferably from 1 to 4), Cs-Fe-Mo mixed bromide salts (with varying Cs/Fe/Mo molar ratios for all 3 elements, preferably from 1 to 4), Li-Fe-Mo mixed bromide salts (with varying Li/Fe/Mo molar ratios for all 3 elements, preferably from 1 to 4), Na-Fe-Mo mixed bromide salts (with varying Na/Fe/Mo molar ratios for all 3 elements, preferably from 1 to 4), K-Fe-Mo mixed bromide salts (with varying K/Fe/Mo molar ratios for all 3 elements, preferably from 1 to 4), Rb-Fe-Mo mixed bromide salts (with varying Rb/Fe/Mo molar ratios for all 3 elements, preferably from 1 to 4), Cs-Fe-Mo mixed phosphate salts (with varying Cs/Fe/Mo molar ratios for all 3 elements, preferably from 1 to 4), Li-Fe-Mo mixed phosphate salts (with varying Li/Fe/Mo molar ratios for all 3 elements, preferably from 1 to 4), Na-Fe-Mo mixed phosphate salts (with varying Na/Fe/Mo molar ratios for all 3 elements, preferably from 1 to 4), K-Fe-Mo mixed phosphate salts (with varying K/Fe/Mo molar ratios for all 3 elements, preferably from 1 to 4), Rb-Fe-Mo mixed phosphate salts (with varying Rb/Fe/Mo molar ratios for all 3 elements, preferably from 1 to 4), Cs-Fe-Mo mixed carbonate salts (with varying Cs/Fe/Mo molar ratios for all 3 elements, preferably from 1 to 4), Li-Fe-Mo mixed carbonate salts (with varying Li/Fe/Mo molar ratios for all 3 elements, preferably from 1 to 4), Na-Fe-Mo mixed carbonate salts (with varying Na/Fe/Mo molar ratios for all 3 elements, preferably from 1 to 4), K-Fe-Mo mixed carbonate salts (with varying K/Fe/Mo molar ratios for all 3 elements, preferably from 1 to 4), Rb-Fe-Mo mixed carbonate salts (with varying Rb/Fe/Mo molar ratios for all 3 elements, preferably from 1 to 4), Cs-Fe-Mo mixed oxalate salts (with varying Cs/Fe/Mo molar ratios for all 3 elements, preferably from 1 to 4), Li-Fe-Mo mixed oxalate salts (with varying Li/Fe/Mo molar ratios for all 3 elements, preferably from 1 to 4), Na-Fe-Mo mixed oxalate salts (with varying Na/Fe/Mo molar ratios for all 3 elements, preferably from 1 to 4), K-Fe-Mo mixed oxalate salts (with varying K/Fe/Mo molar ratios for all 3 elements, preferably from 1 to 4), Rb-Fe-Mo mixed oxalate salts (with varying Rb/Fe/Mo molar ratios for all 3 elements, preferably from 1 to 4).

Preferred catalysts are based on the ZSM-5 aluminosilicate zeolite promoted with Cs-Fe-Mo mixed nitrate salts.

The suitable amount of transition metal in the catalysts of the present invention is in the range of from 1 to 20 weight percent relative to zeolite support. The following examples merely illustrate the invention.

It is to be understood that the preferred embodiments described for the catalyst as such of course also are applicable to the use of the catalyst in the process of the present invention.

Examples of suitable tri-metallic promoted aluminosilicate zeolite catalysts are xLiyFezMo-zeolite, xNayFezMo-zeolite, xKyFezMo-zeolite, xRbyFezMo-zeolite, xCsyFezMo-zeolite, preferably xCsyFezMo-zeolite, wherein the metals are supported on a ZSM-5, Mordenite, Zeolite USY, Zeolite L, Ferrierite, Zeolite Beta, Zeolite Y, wherein most preferably ZSM-5. The alkali and transition metals to be employed in accordance with the present invention are preferably selected among oxide, nitrate, sulfate, chloride, phosphate, carbonate, iodide, bromide, fluoride, oxalate salts, wherein most preferably nitrate salts.

The catalysts in accordance with the present invention preferably are aluminosilicate zeolite ZSM-5 and cesium-iron-molybdenum metals supported on this material. The suitable amount of metals in the zeolite support of the present invention is in the range of from 1 to 20 weight percent relative to the support.

The catalysts disclosed herein, in particular, the xCsyFezMo-zeolite catalytic system disclosed herein has particularly shown to efficiently gas-phase glycerol conversion in a fixed-bed reactor. Without being bound to the following explanation, it may be that the superior and unexpected activity is attributed to the presence of redox and bifunctional acidic-basic sites, and synergetic interaction between Cs, Fe, and Mo species and zeolite support. The suitable balance between acidity-basicity and reducibility behaviors of the catalyst samples are the key parameter to the high selectivity for the desired allyl alcohol production. In any case, the xCsyFezMo-ZSM-5 zeolite catalytic system disclosed here shows very high activity, selectivity and yield of AAL from GL in a gas-phase fixed-bed reactor - higher than other catalysts tested or reported in the literature for the gas-phase conversion of GL to AAL. The heterogenous catalyst described herein is suitable to provide conversion of the GL of more than 98 mol% and a yield of AAL of above 45 mol% in the production of AAL from GL in the gas-phase fixed-bed continuous flow reactor.

In order to demonstrate the superiority of the present invention, these following catalysts have been evaluated in relation with the catalytic conversion of GL to AAL, including ZSM-5 and Cs-Fe-Mo mixed nitrate salts (with varying Cs/Fe/Mo molar ratios, preferably from 1 to 5), with preferred metal ranging from 1 to 20 weight percent relative to the zeolite support. The respective results, some of which are discussed further in relation with experimental data shown below, prove that the catalysts in accordance with the present invention enable the preparation of the desired target molecule in a highly effective manner, thereby overcoming the drawbacks associated with the prior art and solving the problems outlined above.

Accordingly the process in accordance with the present invention comprises the step of converting a starting material, selected glycerol (GL) solution (preferably aqueous solution), preferably obtained from renewable resources (bio-based) to AAL in the presence of a heterogeneous catalyst as defined above, preferably selected among catalysts based on zeolite, preferably selected among ZSM-5, Mordenite, Zeolite USY, Zeolite L, Ferrierite, Zeolite Beta, Zeolite Y, preferably ZSM-5, as well as alkali and transition metals, wherein the alkali and transition metals are supported on a ZSM-5, preferably selected among those exemplified above. As for the catalyst as such the alkali and transition metals to be employed in accordance with the present invention are preferably selected among Li, Na, K, Rb, Cs with Fe and Mo, most preferably Cs with Fe and Mo. These metals preferably selected among oxide, nitrate, sulfate, chloride, phosphate, carbonate, iodide, bromide, fluoride, oxalate salts, wherein preferably nitrate salts.

The starting materials to be converted in accordance with the present invention typically are provided in a polar solvent, preferably water. It has been found that the concentration of the starting material in the polar solvent preferably is at least 1 wt% and not more than 60 wt%, preferably 10 to 35 wt%. It has been found that the amount of catalyst in the gas-phase reaction in fixed-bed continuous flow reactor is not less than 0.5 g and not higher than 5.0 g.

The gas hourly space velocity is in the range from 100 to 2000 $h^{-1}$, preferably from 300 to 1500 $h^{-1}$ and more preferably from 500 to 1000 $h^{-1}$, and most preferably 600-650 $h^{-1}$.

A preferred combination of reaction conditions is a temperature of from 330 to 360 °C and a gas hourly space velocity of from 600 to 650 $h^{-1}$.

Preferably, the reaction is carried out with a carrier gas, and nitrogen gas is a preferred example of a suitable carrier gas, due to its abundance and low cost.

The process disclosed here typically is carried out at a temperature of from 250 to 450 °C, preferably from 300 to 350 °C, more preferably from 330 to 360 °C, such as 350 °C.

The reaction may be carried out in any suitable reactors known to the skilled person, but stainless steel reactors (fixed-bed continuous flow reactor) are in particular suitable.

It is to be understood, that the various process parameters, such as starting material concentration, gas-hourly space velocity, catalyst amount, temperature, while being disclosed individually, are considered in the context of the present invention in combination, such as a process employing water as solvent, a starting material concentration of from 1 to 60 wt%, a catalyst amount of from 0.5 to 5.0 g, a reaction temperature of from 250 to 450 °C, preferably from 330 to 360 °C, and a the gas hourly space velocity of from 100 to 2000 $h^{-1}$. However, all other combinations possible from the ranges and preferred embodiments are also comprised by and disclosed in this invention.

In the examples described herein, the catalytic conversion of glycerol was performed using a stainless steel fixed-bed continuous flow reactor under the standard operation conditions. The novel process of the present invention was carried out at a temperature greater than 250 °C, most preferably between 250 and 450 °C, especially between 330 and 360°C. The reactions were carried out under atmospheric pressure. In general, the reaction should be conducted under conditions where the gas hourly space velocity of the feedstock solution over the catalyst is appropriate to generate the desired products. Preferably, the gas hourly space velocity is in the range of between 100 and 2000 $h^{-1}$, typically 300-1500 $h^{-1}$, more preferably 500-1000 $h^{-1}$, most preferably 600-650 $h^{-1}$. The heterogenous catalyst described herein is suitable to provide conversion of the GL of more than 98 mol% and a yield of AAL of above 45 mol% in the production of AAL from GL in the gas-phase fixed-bed continuous flow reactor.

## EXAMPLES

### EXAMPLE 1

### Materials used

In the next examples, the following starting materials were used: ZSM-5, iron(III) nitrate (Sigma-Aldrich, 98+%), ammonium heptamolybdate tetrahydrate (Sigma-Aldrich, 99.98%), cesium nitrate (Sigma-Aldrich, 99%), glycerol (Sigma-Aldrich, 99%), nitrogen (5.0, Messer, Bad Soden am Taunus, Germany), $NH_3$ (10 vol% in He, Linde, Pullach, Germany), helium (5.0, Messer, Bad Soden am Taunus, Germany) and carbon dioxide (5 vol% CO in He, Linde, Pullach, Germany), glycidol (Sigma-Aldrich, 96%), ethanol (Sigma-Aldrich, >99.8%), acrolein (Fluka, >95%), allyl alcohol (Sigma-Aldrich, >99%), hydroxyacetone (Alfa Aesar, 95%), 2-methoxy-1,3-dioxalane (Sigma-Aldrich, >99%), glycerol formal (Sigma-Aldrich, 98%), acetaldehyde (Sigma-Aldrich, >99.5%), propionaldehyde (Sigma-Aldrich, 97%), acetic acid (Sigma-Aldrich, >99.7%), acetone (Sigma-Aldrich, >99.5%), propionic acid (Sigma-Aldrich, >99.5%), acrylic acid (Sigma-Aldrich, 99%), 1,2-propanediol (Sigma-Aldrich, >99%), n-propanol (Sigma-Aldrich, 99.5%), formaldehyde (Sigma-Aldrich, 37wt% in $H_2O$), methanol (Sigma-Aldrich, >99.9%), ethanol (Sigma-Aldrich, >99.8%). All these materials were used as received from the suppliers, i.e., without any additional purification.

**EXAMPLE 2**

**Catalysts preparation**

The preparation of the xCsyFezMo-ZSM-5 catalysts has involved the impregnation of ZSM-5 zeolite support using the incipient wetness impregnation method with solutions of ammonium heptamolybdate $(NH_4)_6Mo_7O_{24}\cdot 4H_2O$, iron(III) nitrate nonahydrate $Fe(NO_3)_3\cdot 9H_2O$, cesium nitrate $CsNO_3$. Samples containing both certain amounts of the molybdenum and iron were prepared by a two-step impregnation procedure, in which the molybdenum phase with concentration 0.001 M was introduced first and iron salts with concentration 0.01 M was last one. The temperature was raised to 80 °C. Stirring continued until suspension turned into a slurry. Then samples were dried overnight at 110 °C and calcined at 500 °C for 6 hours. Samples containing Cs, Fe and Mo species was prepared by the same procedure above but with adding $CsNO_3$ salt with a concentration of 0.01 M.

**EXAMPLE 3**

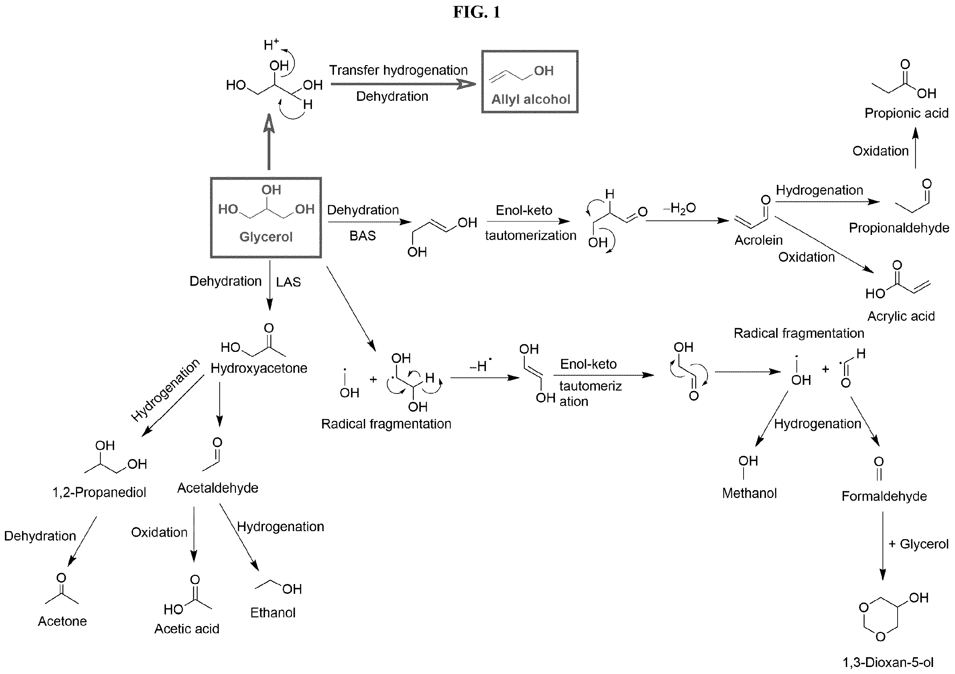

On the Fig. 1 is proposed reaction pathway of AAL formation from GL. The reaction pathway for glycerol conversion into allyl alcohol is a sequence of consecutive and/or parallel dehydration, dehydrogenation, oxidation, transfer hydrogenation reactions and radical decomposition with the participation of acid, base, and redox sites. With utilizing the xCsyFezMo-ZSM-5 catalysts, we have succeeded in producing of allyl alcohol as the main reaction liquid product in a gas-phase fixed-bed reactor. Hydroxyacetone, acrolein, 1,2-propanediol, acetaldehyde, acetone, acetic acid, ethanol, 1,3-dioxan-5-ol, formaldehyde, methanol, acrylic acid, propionaldehyde, and propionic acid were the remaining identified byproducts. We can speculate that allyl alcohol was obtained directly from glycerol over basic sites of CsFeMo-ZSM-5 catalyst through the dehydration and transfer hydrogenation mechanism. The hydrogen donor can act from glycerol and/or water.

**EXAMPLE 4**

**Catalytic evaluation**

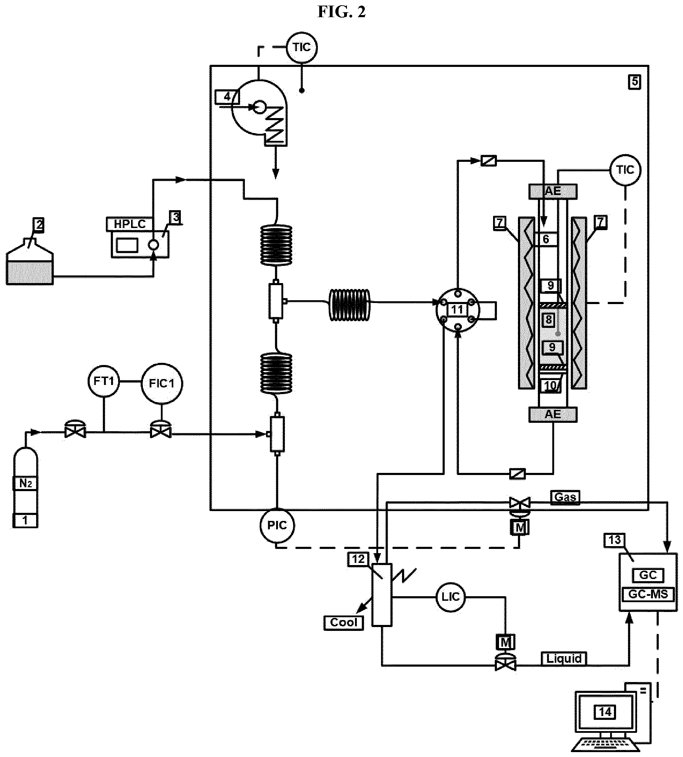

The gas-phase conversion of GL into AAL (Fig. 2) was studied in an automated laboratory fixed-bed microactivity reactor (Microactivity-Reference, PID Eng&Tech). 0.5 g of the catalyst was placed in a packed bed tubular reactor and stabilized with glass wool on both sides. The glycerol 10 wt% aqueous solution was kept at room temperature in a reservoir tank (3) outside of the hot box. It was injected into the hot box with a 307 HPLC Piston Pump (4) (Gilson Inc.). The $N_2$ gas was introduced into the hot box (6) with mass flow controllers (FT1). The glycerol aqueous solution was entered to the hot box (6) was vaporized, since the inside temperature was held at 140 °C, with the help of an electric convection heater (5), controlled by the temperature controller, TIC. After it was mixed of the glycerol solution vapour and was introduced $N_2$ gas. The inlet stream was then passed through a six-port VICI reactor standard bypass valve (12) and was either introduced into the reactor (7) or sent out of the reactor. The reactor was a stainless steel tube (9 mm internal diameter, 305 mm long). The catalyst was packed inside of the tube (9) and insulated with a 15 mm layer of glass wool at the top and bottom (10). The temperature of the packed-bed was measured with a thermocouple with the TIC controller. After leaving the reactor tube, the outlet stream was conducted through the six-port bypass valve (12) and into the Peltier cell (13). The product was cooled down inside the internal storage. The liquid level in the storage was controlled with a level controller (LIC) and the excess product was sent into absorber placed below the reactor.

The collected liquid samples were then analyzed by gas chromatography GC (Agilent GC-7890A) with measurement repeatability of the relative standard deviation (RSD) ≤ 5% equipped with FID detector. Hydrogen used as carrier gas with a flow rate of 1 mL/min. The capillary column was 30m × 0.25mm × 0.25$\mu$m DB-WAX Ultra Inert. To achieve effective product separation, the column was held at 40 °C for 4 min before the temperature was ramped up to 200 °C at a rate of 12 °C/min and remained there for 10 min. The split ratio was 1:150. All products were quantified via an external calibration method verified by a gas chromatography-mass spectrometry system (Shimadzu, GC/MS-QP2010) with the capillary column 30m × 0.25mm × 0.5um Zebron ZB-WAX-Plus.

Calibration curves were measured in the 0.1-10 wt% range by using chemicals mentioned above in the chemical used paragraph. The conversion of glycerol, $X_{GL}$, and the selectivity to the product i, S$i$, calculated using the equations below, where $n$ refers to the moles of compound and in/out to the reactor inlet/outlet.

$$X_{GL}\left(mol\%\right) = \frac{\left(n_{GL}^{in} - n_{GL}^{out}\right)}{n_{GL}^{in}} \times 100 \; ;$$

$$S_i \left( mol\% \right) = \frac{n_i}{n_{GL}^{in} - n_{GL}^{out}} \times \frac{Z_i}{Z_{GL}} \times 100 \; ;$$

$$Y_i \left( mol\% \right) = \frac{n_i}{n_{GL}^{in}} \times \frac{Z_i}{Z_{GL}} \times 100 \; ;$$

where $n_{GL}^{in}$ and $n_{GL}^{out}$ are the input and output flows of glycerol (mol/min), $n_i$ is the flow of the products i (mol/min), $Z_{GL}$ = 3 (the number of carbon atoms in the GL molecule), and $Z_i$ represents the number of carbon atoms in the products. The values in this article are in mole percent. The mass balance was calculated for each experiment and was usually above 92%, where the difference in mass balance was in the range of the experimental error. The studied conversion and selectivity figures were calculated by the values of three measurements.

**EXAMPLE 5**

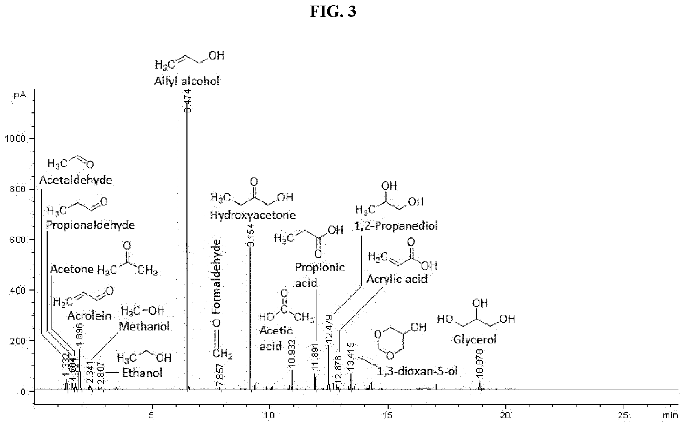

GC-FID traces for a typical run over the 5Cs2.5Fe2.5Mo-ZSM-5 catalyst shows on the Fig. 3 the formation of AAL, hydroxyacetone, 1,2-propanediol, acrolein, acetaldehyde, propionaldehyde, acetone, methanol, ethanol, acetic acid, acrylic acid, propanoic acid, and 1,3-dioxan-5-ol which are the main reaction liquid products, with approximately 91% of total mass.

**EXAMPLE 6**

**Catalytic conversion of GL to AAL using xCsyFezMo-ZSM-5 catalysts**

The conversion of GL to AAL has been performed in the same conditions as those described in the example above (Catalyst evaluation paragraph). The results obtained with over of the catalysts of formula xCsyFezMo-ZSM-5 are given in Table 1 below:

**Table 1.** Gas-phase glycerol conversion into allyl alcohol through a hydrogen transfer reaction.

| Catalyst | Allyl alcohol yield (mol%) | Glycerol conversion (mol%) | Allyl alcohol selectivity (mol%) | Time-on-stream (h) |
|---|---|---|---|---|
| 2.5Fe2.5Mo-ZSM-5 | 16.6 | 97.9 | 17.0 | 24 |
| 2.5Cs2.5Fe2.5Mo-ZSM-5 | 39.9 | 98.2 | 40.6 | 23 |
| 5Cs2.5Fe2.5Mo-ZSM-5 | 45.2 | 98.1 | 46.1 | 26 |
| 10Cs2.5Fe2.5Mo-ZSM-5 | 48.8 | 93.8 | 52.0 | 6 |

**EXAMPLE 7**

Catalytic conversion of GL to AAL using xCsyFezMo-ZSM-5 catalysts and comparison with $Fe_2O_3$, $ZrO_2$-FeOx, K-$ZrO_2$-FeOx, Fe-γ-alumina, Fe-Rb-γ-alumina, 13wt%Fe-Rb-ZSM-5, and 3.84wt%Fe1.16wt%Mo-KIT-6 catalysts. The results are given in the following Table 2.

**Table 2.** A comparison of existing routes of gas-phase glycerol conversion into allyl alcohol through a hydrogen transfer reaction with the present work.

| Catalyst | Allyl alcohol yield (mol%) | TOS (h) | $T_{reaction}$ ($^0$C) | $C_{GL}$ (wt%) | $m_{cat}$ (g) | Ref. |
|---|---|---|---|---|---|---|
| $Fe_2O_3$ (*) | 20 | 24 | 320 | 35 | 0.15 | (Liu et al., 2010) |

(continued)

| Catalyst | Allyl alcohol yield (mol%) | TOS (h) | $T_{reaction}$ ($^0$C) | $C_{GL}$ (wt%) | $m_{cat}$ (g) | Ref. |
|---|---|---|---|---|---|---|
| ZrO$_2$-FeOx (*) | 20 | 2 | 350 | 10 | N/A | (Yoshikawa et al., 2011) |
| K-ZrO$_2$-FeOx (*) | 27 | 6-8 | 350 | 10-50 | N/A | (Konaka et al., 2014, 2013) |
| Fe-$\gamma$-alumina (*) | 4.0 | 3 | 340 | 35 | 5.00 | ( Sanchez et al., 2014) |
| Fe-Rb-$\gamma$-alumina (*) | 11.6 | 3 | 340 | 35 | 5.00 | |
| 13Fe-Rb-ZSM-5 (*) | 11.8 | 6 | 340 | 35 | 0.5-1.0 | (Sanchez et al., 2016) |
| 3.84Fe1.16Mo-KIT-6 (*) | 26.8 | 2 | 340 | 35 | 0.20 | (Lan et al., 2017) |
| FeMo/CeO$_2$ | 22.6 | 2 | 340 | 35 | 0.20 | (Lan et al., 2019) |
| 5Cs2.5Fe2.5Mo-ZSM-5 | 45.2 | 26 | 350 | 10 | 0.50 | Invention |
| (*) Comparative catalyst not forming part of the invention | | | | | | |

These results demonstrate that the preferred 5Cs2.5Fe2.5Mo-ZSM-5 catalyst has a superior catalytic activity compared to the other well-known catalysts.

**Claims**

1. Process for the production of allyl alcohol (AAL) comprising the steps:

    (a) feeding a starting material into a fixed-bed continuous flow reactor over a heterogeneous trimetallic aluminosilicate zeolite catalyst comprising varying amounts of Cs-Fe-Mo, Li-Fe-Mo, Na-Fe-Mo, K-Fe-Mo or Rb-Fe-Mo, wherein these metals can be present as mixed oxides, mixed nitrate salts, mixed fluoride, chloride, bromide or iodide salts, mixed sulfate salts, mixed phosphate salts, mixed carbonate salts or mixed oxalate salts,
    (b) heating said mixture at a temperature ranging from 250 to 450 °C to obtain allyl alcohol,
    (c) flowing N$_2$ as a carrier gas with total gas hourly space velocity 100-2000 h$^{-1}$,
    (d) collecting the AAL product,

    wherein the starting material is glycerol (GL) solution.

2. The process as claimed in claim 1, wherein the molar ratios for all 3 metals vary from 1 to 4.

3. The process as claimed in any one of the preceding claims, wherein the zeolite is selected from the group consisting of ZSM-5, Mordenite, Zeolite USY, Zeolite L, Ferrierite, Zeolite Beta, and Zeolite Y.

4. The process according to any one of the preceding claims, wherein the heterogenous catalyst is xLiyFezMo-zeolite, xNayFezMo-zeolite, xKyFezMo-zeolite, xRbyFezMo-zeolite, or xCsyFezMo-zeolite, wherein x, y, and z represent weight loading of metal species,
    preferably wherein the catalyst is the xCsyFezMo-ZSM-5 aluminosilicate zeolite (B01J29/061), where x, y, and z represent weight loading of metal species.

5. The process as claimed in any one of the preceding claims, wherein the metals are present in an amount of 1 to 20 wt% relative to zeolite support.

6. The process as claimed in any one of the preceding claims, wherein the mixture further comprises water.

7. The process as claimed in at least one of the preceding claims, wherein the amount of the catalysts consisting of an aluminosilicate zeolite carrier and three metals ranges from 0.5 to 5 g.

8. The process as claimed in any one of the preceding claims, wherein the concentration of GL is in the range from 1 to 60 wt%.

9. The process according to any one of the preceding claims, wherein the temperature is from 250 °C to 450 °C.

10. The process according to any one of the preceding claims, wherein the gas hourly space velocity is from 100 to 2000 h$^{-1}$.

11. A heterogenous trimetallic aluminosilicate zeolite catalyst comprising varying amounts of Cs-Fe-Mo, Li-Fe-Mo, Na-Fe-Mo, K-Fe-Mo or Rb-Fe-Mo, wherein these metals can be present as mixed oxides, mixed nitrate salts, mixed fluoride, chloride, bromide or iodide salts, mixed sulfate salts, mixed phosphate salts, mixed carbonate salts or mixed oxalate salts, for the production of AAL from GL.

12. The catalyst as claimed in claim 11, wherein the molar ratios for all 3 metals vary from 1 to 4.

13. The catalyst as claimed in any one claims 11-12, wherein the zeolite is selected from the group consisting of ZSM-5, Mordenite, Zeolite USY, Zeolite L, Ferrierite, Zeolite Beta, and Zeolite Y.

14. The catalyst as claimed in any one of claims 11-13, which is xLiyFezMo-zeolite, xNayFezMo-zeolite, xKyFezMo-zeolite, xRbyFezMo-zeolite, or xCsyFezMo-zeolite, wherein x, y, and z represent weight loading of metal species, preferably wherein the catalyst is the xCsyFezMo-ZSM-5 aluminosilicate zeolite, where x, y, and z represent weight loading of metal species.

15. The catalyst as claimed in any one of claims 11-14, wherein the metals are present in an amount of 1 to 20 wt% relative to zeolite support.

## Patentansprüche

1. Verfahren zur Herstellung von Allylalkohol (AAL), umfassend die Schritte:

   (a) Zuführen eines Ausgangsmaterials in einen kontinuierlichen Festbettdurchflussreaktor über einen heterogenen trimetallischen Aluminosilicat-Zeolith-Katalysator, der variierende Mengen an Cs-Fe-Mo, Li-Fe-Mo, Na-Fe-Mo, K-Fe-Mo oder Rb-Fe-Mo umfasst, wobei diese Metalle als gemischte Oxide, gemischte Nitratsalze, gemischte Fluorid-, Chlorid- oder Iodid-Salze, gemischte Sulfatsalze, gemischte Phosphatsalze, gemischte Carbonatsalze oder gemischte Oxalatsalze vorliegen können,
   (b) Erwärmen der Mischung auf eine Temperatur im Bereich von 250 bis 450°C um Allylalkohol zu erhalten,
   (c) Strömen von N$_2$ als ein Trägergas mit einer stündlichen Gesamtgasraumgeschwindigkeit von 100-2000 h$^{-1}$,
   (d) Sammeln des AAL Produkts,

   wobei das Ausgangsmaterial eine Glycerol- (CL) Lösung ist.

2. Verfahren wie in Anspruch 1 beansprucht, wobei die Molverhältnisse für alle 3 Metalle von 1 bis 4 variieren.

3. Verfahren wie in einem der vorhergehenden Ansprüche beansprucht, wobei der Zeolith ausgewählt ist aus der Gruppe bestehend aus ZSM-5, Mordenit, Zeolith USY, Zeolith L, Ferrierit, Zeolith Beta und Zeolith Y.

4. Verfahren nach einem der vorhergehenden Ansprüche, wobei der heterologe Katalysator xLiyFezMo-Zeolith, xNay-FezMo-Zeolith, xKyFezMo-Zeolith, xRbyFezMo-Zeolith oder xCsyFezMo-Zeolith ist, wobei x, y und z eine Gewichtsbeladung an Metallspezies darstellen,
   vorzugsweise wobei der Katalysator der xCsyFezMo-ZSM-5 Aluminosilicat-Zeolith (B01J29/061) ist, wobei x, y und z eine Gewichtsbeladung an Metallspezies darstellen.

5. Verfahren wie in einem der vorhergehenden Ansprüche beansprucht, wobei die Metalle in einer Menge von 1 bis 20 Gew.-% in Bezug auf Zeolith-Träger vorliegen.

**6.** Verfahren wie in einem der vorhergehenden Ansprüche beansprucht, wobei die Mischung ferner Wasser umfasst.

**7.** Verfahren wie in mindestens einem der vorhergehenden Ansprüche beansprucht, wobei die Menge der Katalysatoren, die aus einem Aluminosilicat-Zeolith-Träger und drei Metallen bestehen im Bereich von 0,5 bis 5 g liegt.

**8.** Verfahren wie in einem der vorhergehenden Ansprüche beansprucht, wobei die Konzentration an GL im Bereich von 1 bis 60 Gew.-% liegt.

**9.** Verfahren nach einem der vorhergehenden Ansprüche, wobei die Temperatur von 250 °C bis 450 °C beträgt.

**10.** Verfahren nach einem der vorhergehenden Ansprüche, wobei die stündliche Gasraumgeschwindigkeit von 100 bis 2000 h$^{-1}$ beträgt.

**11.** Heterologer trimetallischer Aluminosilicat-Zeolith-Katalysator, umfassend variierende Mengen an Cs-Fe-Mo, Li-Fe-Mo, Na-Fe-Mo, K-Fe-Mo oder Rb-Fe-Mo, wobei diese Metalle als gemischte Oxide, gemischte Nitratsalze, gemischte Fluorid-, Chlorid-, Bromid- oder Iodidsalze, gemischte Sulfatsalze, gemischte Phosphatsalze, gemischte Carbonatsalze oder gemischte Oxalatsalze vorliegen können, zur Herstellung von AAL aus GL.

**12.** Katalysator wie in Anspruch 11 beansprucht, wobei die Molverhältnisse für alle 3 Metalle von 1 bis 4 variieren.

**13.** Katalysator wie in einem der Ansprüche 11-12 beansprucht, wobei der Zeolith ausgewählt ist aus der Gruppe bestehend aus ZSM-5, Mordenit, Zeolith USY, Zeolith L, Ferrierit, Zeolith Beta und Zeolith Y.

**14.** Katalysator wie in einem der Ansprüche 11-13 beansprucht, der xLiyFezMo-Zeolith, xNayFezMo-Zeolith, xKyFezMo-Zeolith, xRbyFezMo-Zeolith oder xCsyFezMo-Zeolith ist, vorzugsweise wobei der Katalysator der xCsyFezMo-ZSM-5 Aluminosilicat-Zeolith ist, wobei x, y und z eine Gewichtsbeladung an Metallspezies darstellen.

**15.** Katalysator wie in einem der Ansprüche 11-14 beansprucht, wobei die Metalle in einer Menge von 1 bis 20 Gew.-% bezogen auf Zeolith-Träger vorliegen.

**Revendications**

**1.** Procédé pour la production d'alcool allylique (AAL) comprenant les étapes de :

(a) alimentation en matière de départ dans un réacteur à flux continu à lit fixe sur un catalyseur de zéolite aluminosilicate trimétallique hétérogène comprenant diverses quantités de Cs-Fe-Mo, Li-Fe-Mo, Na-Fe-Mo, K-Fe-Mo ou Rb-Fe-Mo, dans lequel ces métaux peuvent être présents en tant qu'oxydes mixtes, sels de nitrate mixtes, sels de fluorure, de chlorure, de bromure ou d'iodure mixtes, sels de sulfate mixtes, sels de phosphate mixtes, sels de carbonate mixtes ou sels d'oxalate mixtes,
(b) chauffage dudit mélange à une température allant de 250 à 450 °C pour obtenir l'alcool allylique,
(c) circulation de $N_2$ en tant que gaz vecteur à une vitesse spatiale horaire gazeuse totale de 100-2000 h$^{-1}$,
(d) de collecte du produit AAL,

dans lequel la matière de départ est une solution de glycérol (GL).

**2.** Procédé selon la revendication 1, dans lequel les rapports molaires pour les 3 métaux varient de 1 à 4.

**3.** Procédé selon l'une quelconque des revendications précédentes, dans lequel la zéolite est sélectionnée dans le groupe constitué de ZSM-5, mordénite, zéolite USY, zéolite L, ferriérite, zéolite bêta, et zéolite Y.

**4.** Procédé selon l'une quelconque des revendications précédentes, dans lequel le catalyseur hétérogène est la xLiyFezMo-zéolite, xNayFezMo-zéolite, xKyFezMo-zéolite, xRbyFezMo-zéolite, ou xCsyFezMo-zéolite, dans lequel x, y, et z représentent la charge pondérale des espèces métalliques, de préférence dans lequel le catalyseur est la zéolite aluminosilicate xCsyFezMo-ZSM-5 (B01J29/061), où x, y, et z représentent la charge pondérale des espèces métalliques.

**5.** Procédé selon l'une quelconque des revendications précédentes, dans lequel les métaux sont présents dans une quantité de 1 à 20 % en poids par rapport au support de zéolite.

6. Procédé selon l'une quelconque des revendications précédentes, dans lequel le mélange comprend en outre de l'eau.

7. Procédé selon au moins l'une des revendications précédentes, dans lequel la quantité des catalyseurs consisitant en un vecteur zéolite aluminosilicate et trois métaux varie de 0,5 à 5 g.

8. Procédé selon l'une quelconque des revendications précédentes, dans lequel la concentration de GL est dans la gamme de 1 à 60 % en poids.

9. Procédé selon l'une quelconque des revendications précédentes, dans lequel la température est de 250 °C à 450 °C.

10. Procédé selon l'une quelconque des revendications précédentes, dans lequel la vitesse spatiale horaire gazeuse est de 100 à 2000 h$^{-1}$.

11. Catalyseur de zéolite aluminosilicate trimétallique hétérogène comprenant diverses quantités de Cs-Fe-Mo, Li-Fe-Mo, Na-Fe-Mo, K-Fe-Mo ou Rb-Fe-Mo, dans lequel ces métaux peuvent être présents en tant qu'oxydes mixtes, sels de nitrate mixtes, , sels de fluorure, de chlorure, de bromure ou d'iodure mixtes, sels de sulfate mixtes, sels de phosphate mixtes, sels de carbonate mixtes ou sels d'oxalate mixtes, pour la production de AAL à partir de GL.

12. Catalyseur selon la revendication 11, dans lequel les rapports molaires pour les 3 métaux varient de 1 à 4.

13. Catalyseur selon l'une quelconque des revendications 11-12, dans lequel la zéolite est sélectionnée dans le groupe constitué de ZSM-5, mordénite, zéolite USY, zéolite L, ferriérite, zéolite bêta, et zéolite Y.

14. Catalyseur selon l'une quelconque des revendications 11-13, qui est la xLiyFezMo-zéolite, xNayFezMo-zéolite, xKyFezMo-zéolite, xRbyFezMo-zéolite, ou xCsyFezMo-zéolite, dans lequel x, y, et z représentent la charge pondérale des espèces métalliques,
de préférence dans lequel le catalyseur est la zéolite aluminosilicate xCsyFezMo-ZSM-5, où x, y, et z représentent la charge pondérale des espèces métalliques.

15. Catalyseur selon l'une quelconque des revendications 11-14, dans lequel les métaux sont présents dans une quantité de 1 à 20 % en poids par rapport au support de zéolite.

**FIG. 1**

FIG. 2

**FIG. 3**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HAI LAN et al.** *Catal Lett,* 2017, vol. 147, 2187-2199